# EUROPEAN PATENT APPLICATION

(11) **EP 4 464 283 A1**
(43) Date of publication of application: **20.11.2024**
(21) Application number: 22918940.2
(22) Date of filing: 04.03.2022
(51) Int. Cl.: A61F 2/70, A61F 2/58, H02K 7/116, A61F 2/68

(54) **STRUCTURE OF MOTOR AND REDUCER BUILT INTO PROSTHETIC FINGER**

(30) Priority: 10.01.2022 KR 20220003444; 28.01.2022 KR 20220013440
(71) Applicant: Mand.Ro Co., Ltd., Bucheon-si, Gyeonggi-do 14557 (KR)
(72) Inventor: YI, Sang Ho, Seoul 05502 (KR)
(74) Representative: Rückerl, Florian
(86) International application number: PCT/KR2022/003116
(87) International publication number: WO 2023/132403

(57) **Abstract**

The present invention relates to a motor and reducer structure built in a prosthetic finger which includes a first phalanx having a shape corresponding to a shape from a distal phalanx to a middle phalanx of a finger and a motor built in the first phalanx. According to the present invention, since an ultra-small motor is built in the first phalanx of the prosthetic finger, the demands of partial hand amputees of which only fingers are amputated can be satisfied, since a reducer is built between a first joint and a second joint, the first joint and the second joint can be synchronized to perform joint movements, a cable operates by being disposed in contact with an outer side of a first outer circumferential surface of the first joint and an inner side of a second outer circumferential surface of the second joint positioned at an opposite side of the first outer circumferential surface, tension or shrinkage of the cable in a longitudinal direction can be prevented, since the motor and the reducer can be built in different phalanxes, a length and a shape similar to those of an actual human finger can be more easily implemented, and thus the prosthetic finger having a shape similar to that of the actual human finger compared to the conventional technology can be provided.

## Description

### [Technical Field]

The present invention relates to a prosthetic finger. More specifically, the present invention relates to a prosthetic finger with a motor and reducer built-in structure.

### [Background Art]

Electronic prosthetic hands are used as a way to treat the disability of partial hand amputees. However, since most conventional electronic prosthetic hands have motors positioned in uninjured palm areas, in most cases, the conventional electronic prosthetic hands cannot be actually used. Since products of fingers in which motors are built developed to improve conventional electronic prosthetic hands are sold at high prices of more than or equal to 10 million won per finger, most partial hand amputees who actually require electronic prosthetic hands do not use the conventional electronic prosthetic hands.

In addition, since the conventional technology improved to build a motor in a finger is developed so that the motor and a reducer are built-in between a proximal interphalangeal (PIP) joint and a metacarpophalangeal joint (MCP) joint, there is a limitation to minimize a length between the PIP joint and the MCP joint. Accordingly, there is a problem of implementing a prosthetic hand having a finger length similar to an actual finger length.

In the present invention, both a motor and a reducer are built in a prosthetic finger but are separately built therein, and a length between a PIP joint and an MCP joint is flexibly adjusted so that the prosthetic finger of which a length is more similar to an actual finger length. In addition, the present invention is provided to reduce manufacturing costs so that partial hand amputees who are in the blind spot of welfare benefits can use an electronic prosthetic hand with improved functions.

### [Patent Document]

Related Document 1: Korean Patent No. 10-0581038 (Registered on May 10, 2006)
Related document 2: Korean Patent No. 10-0895872 (Registered on April 24, 2009)

### [Disclosure]

### [Technical Problem]

The present invention is directed to providing an electronic prosthetic hand in which an ultra-small motor is built in the finger thereof to reduce a manufacturing cost and which is improved to satisfy needs of an actual user.

The present invention is directed to providing an electronic prosthetic hand in which a motor and a reducer are built in different phalanx portions to flexibly adjust a length of each phalanx.

### [Technical Solution]

One aspect of the present invention provides a prosthetic finger including a first phalanx having a shape corresponding to a shape of a distal phalanx to a middle phalanx of a finger and a motor built in the first phalanx.

### [Advantageous Effects]

According to the present invention, since an ultra-small motor is built in a first phalanx of a prosthetic finger, the demands of partial hand amputees of which only fingers are amputated can be satisfied.

According to the present invention, since a reducer is built between a first joint and a second joint, the first joint and the second joint can be synchronized to perform joint movements.

According to the present invention, since a cable operates by being disposed in contact a first outer circumferential surface of a first joint and a second outer circumferential surface of a second joint, the tension or shrinkage of the cable in a longitudinal direction can be prevented.

According to the present invention, unlike the conventional technology, since a motor structure and a reducer structure are divided and disposed in two different phalanxes of a finger, an electric prosthetic hand having a length more similar to a length of a human finger can be provided.

### [Description of Drawings]

FIG. 1 shows front and side views of a prosthetic finger according to one embodiment of the present invention.
FIG. 2 shows cross-sectional views of the prosthetic finger according to one embodiment of the present invention.
FIG. 3 is a cross-sectional view illustrating an operation of a cable in the prosthetic finger according to one embodiment of the present invention.

### [Best Modes of the Invention]

A prosthetic finger including a first phalanx having a shape corresponding to a shape from a distal phalanx to a middle phalanx of a finger and a motor built in the first phalanx is provided.

### [Modes of the Invention]

The purposes and effects of the present invention will become clearer through the following detailed description, but the purposes and effects of the present invention are not limited to the following description. In addition, in describing the present invention, the detailed descriptions of well-known technologies related to the present invention that unnecessarily obscure the gist of the present invention will be omitted.

Hereinafter, embodiments of the present invention will be described in detail with reference to the accompanying drawings in order for those skilled in the art to easily perform the present invention. The present invention may be implemented in several different forms and is not limited to the embodiments described herein. In addition, portions irrelevant to the description of the present invention are omitted in the drawings in order to clearly describe the present invention, and the same or similar components are denoted by the same reference numerals.

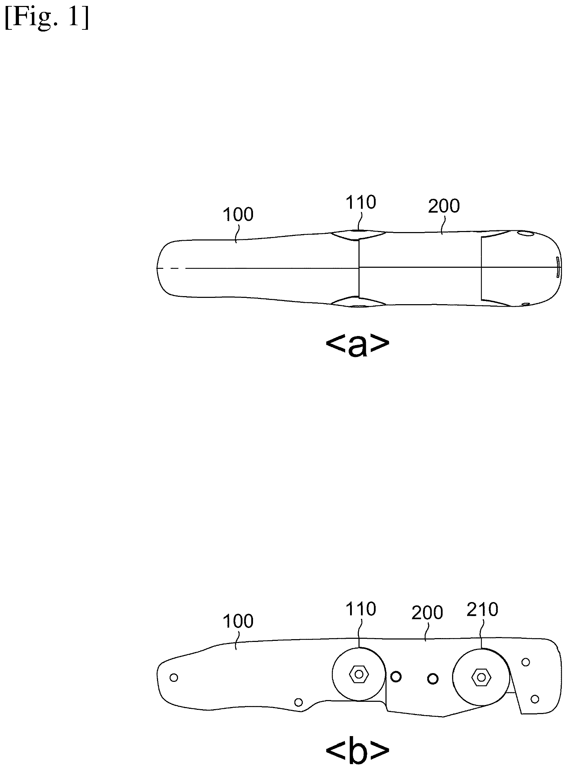

FIG. 1 shows front and side views of a prosthetic finger according to one embodiment of the present invention. Specifically, FIG. 1A is the front view of the prosthetic finger according to one embodiment of the present invention, and FIG. 1B is the side view of the prosthetic finger according to one embodiment of the present invention.

Referring to FIG. 1, the prosthetic finger according to one embodiment of the present invention includes a first phalanx 100, a second phalanx 200, a first joint 110, and a second joint 210.

Specifically, the first phalanx 100 is a phalanx which has a shape from a distal phalanx to a middle phalanx of a finger, and the second phalanx 200 is phalanx which has a shape of a phalanx of the finger connected to a palm. In addition, the first joint 110 is a joint which connects the first phalanx 100 and the second phalanx 200 to perform a first joint movement, and the second joint 210 is a joint which connects the second phalanx 200 and the palm to perform a second joint movement.

More specifically, the first joint 110 is a joint which connects one end of the second phalanx 200 (which is an end formed on the left side in FIG. 1) and the other end of the first phalanx 100 (which is an end formed on the right side in FIG. 1) to perform a joint movement and serves a function corresponding to a proximal interphalangeal (PIP) joint in an actual human finger. In addition, the second joint 210 is a joint which is formed at the other end of the second phalanx 200 to perform a joint movement and serves a function corresponding to a metacarpophalangeal (MCP) joint in the actual human finger. In this case, the first joint 110 and the second joint 210 are provided to receive power from a motor M, which will be described below, to perform the joint movements and operate in synchronization with each other by a reducer G which will be described below.

As another exemplary embodiment, the prosthetic finger according to the present invention may include the first phalanx 100 divided into two portions having shapes corresponding to the distal phalanx and the middle phalanx of the finger and may further include a joint formed to connect the two portions. That is, the prosthetic finger may be provided to further include the joint corresponding to the DIP joint in the actual human finger.

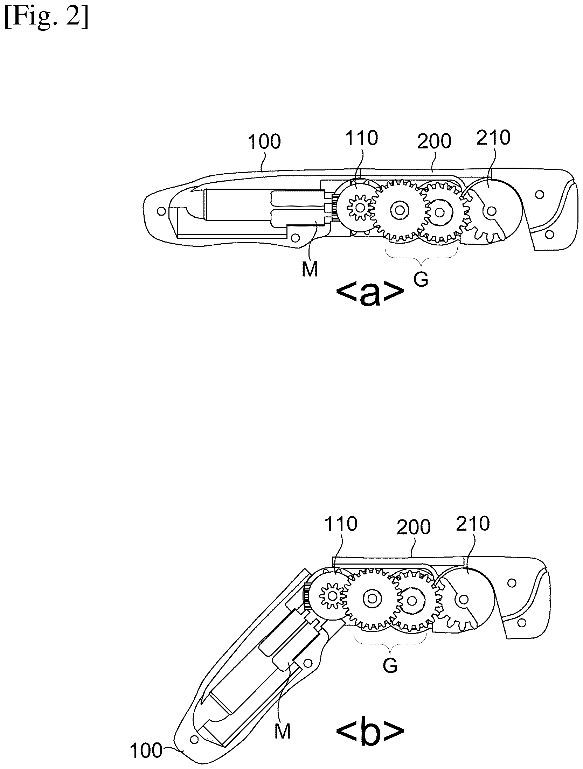

FIG. 2 shows cross-sectional views of the prosthetic finger according to one embodiment of the present invention. Specifically, FIG. 2A is a cross-sectional view of the prosthetic finger according to one embodiment of the present invention, and FIG. 2B is a cross-sectional view of the prosthetic finger performing joint movement according to one embodiment of the present invention.

Referring to FIG. 2, the prosthetic finger according to one embodiment of the present invention further includes a motor M and a reducer G. Specifically, the prosthetic finger according to one embodiment of the present invention is provided to build the motor M in the first phalanx 100.

In one specific embodiment, it may be most desirable that the motor M built in the prosthetic finger according to one embodiment of the present invention is provided as an ultra-small brushless DC motor M to minimize power loss and noise generation and improve durability. In addition, a control circuit may be disposed on a side surface or upper and lower surfaces of the motor M to control the motor M, and the first joint 110 may be provided as a crown gear to serve a function of transmitting the power of the motor M.

As a more exemplary embodiment, the prosthetic finger according to one embodiment of the present invention may be provided to build the reducer G in the second phalanx 200. Specifically, the reducer G is disposed in the second phalanx 200 to be positioned between the first joint 110 and the second joint 210. The reducer G built in the second phalanx 200 has a multi-stage complex gear structure for deceleration, and as a result of the deceleration performed by the reducer G, the first joint 110 and the second joint 210 are synchronized with each other to perform the joint movement. Since the reducer G with the multi-stage complex gear structure is disposed between the first joint 110 and the second joint 210, the power of the motor M is transmitted to each joint through the reducer G, and thus both joints can be driven with strong durability.

As described above, in the prosthetic finger according to one embodiment of the present invention, since the motor M and the reducer G are disposed separately in the first phalanx 100 and the second phalanx 200, respectively, the second phalanx 200 may be manufactured so that a length of the second phalanx 200 is flexibly adjusted according to the number of gears of the reducer G. This overcomes a limit that the previously disclosed prosthetic fingers have a motor and a reducer built in the same phalanx having a length only for accommodating the motor and the reducer. Accordingly, a prosthetic finger having a phalanx of which a length is similar to that of an actual finger can be manufactured.

In this case, as another embodiment of the motor M, a gearbox which serves to perform deceleration at a lower decelerating ratio than the reducer G may be built in the motor M built in the prosthetic finger according to one embodiment of the present invention. Specifically, a gearbox having a decelerating ratio in the range of about 1:20 to 1:50 may be built. When the gearbox is built in the motor M as described above, since the deceleration performed by the reducer G may be shared and served, there is an effect of improving performance and durability. Specifically, the gearbox built in the motor M may serve to perform deceleration at a low torque and a high speed, and the reducer G may serve to perform deceleration at a high torque and a low speed. However, the gearbox built in the motor M is not limited to the present embodiment and may have the same decelerating ratio as the reducer G or a higher decelerating ratio than the reducer G.

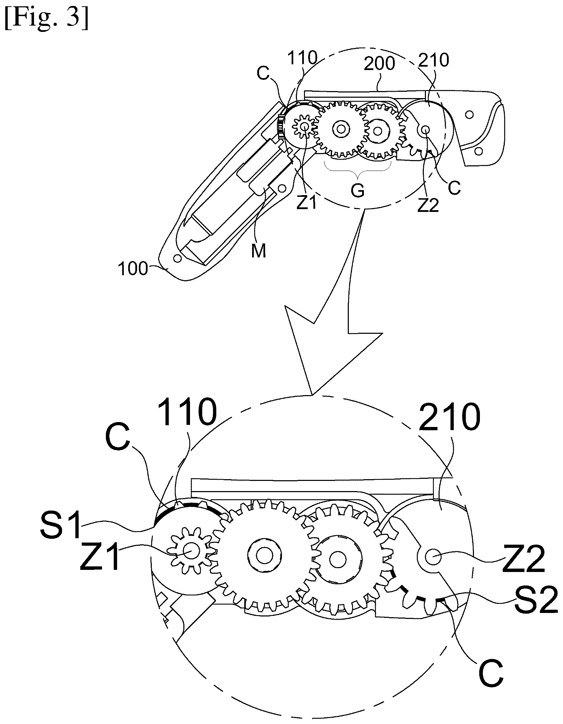

FIG. 3 is a cross-sectional view illustrating an operation of a cable in the prosthetic finger according to one embodiment of the present invention. Referring to FIG. 3, the prosthetic finger according to one embodiment of the present invention may be provided to further include a cable C for transmitting the power of the motor M to the first joint 110 and the second joint 210.

Specifically, the cable C is provided to pass through the first joint 110 and the second joint 210 for supplying power to and controlling the motor M. In this case, the cable C moves along a first outer circumferential surface S1 of the first joint 110 and a second outer circumferential surface S2 of the second joint 210. Specifically, the cable C operates by being disposed in contact with the first outer circumferential surface S1 of the first joint 110 positioned above a first rotary shaft Z1 of the first joint 110 and the second outer circumferential surface S2 of the second joint 210 positioned below a second rotary shaft Z2 of the second joint 210. As a result, the cable C transmits the power of the motor M to the first joint 110 and the second joint 210 and operates so that a tensile force caused by the joint movements of the first joint 110 and the second joint 210 is offset. Specifically, when bending occurs due to the first joint movement of the first joint 110, a path of the cable C increases, and when bending occurs due to the second joint movement of the second joint 210, a path of the cable C decreases. Accordingly, it may be desirable to form a structure which mutually offsets changes in the paths caused by the bending.

As another embodiment, the cable C may operate by being disposed in contact with a third outer circumferential surface of the first joint 110 positioned below the first rotary shaft Z1 of the first joint 110 and a fourth outer circumferential surface of the second joint 210 positioned above the second rotary shaft Z2.

As described above, the cable C of the prosthetic finger according to the present invention is not limited to the structure in which the cable C is disposed in contact with the first outer circumferential surface S1 and the second outer circumferential surface S2 as illustrated in FIG. 3, and the cable C may be formed with a structure in which the cable C is disposed in contact with the outer circumferential surfaces which are symmetrical in a vertical direction with respect to the central axis of each of the first joint 110 and the second joint 210 to offset the tension in a longitudinal direction of the cable C occurred due to the bending.

The above-described exemplary embodiments of the present invention are disclosed to exemplify the present invention and may be variously changed, modified, and added by those skilled in the art within the spirit and scope of the present invention, and such changes, modifications, and additions will fall within the range of the scope. In addition, since such changes, modifications, and additions may be made by those skilled in the art without departing from the technical spirit of the present invention, the present invention is not limited to the above-described description and the accompanying drawings.

In the above-described exemplary system, the methods have described based on a flowchart as a series of steps or blocks, however, the present invention is not limited to the order of the steps, and some steps may occur simultaneously or in an order and steps which are different from those described above. In addition, those skilled in the art may understand that the steps described in the flowchart are not exclusive, other steps may be included, or one or more steps in the flowchart may be deleted without affecting the scope of the present invention.

### [Industrial Applicability]

According to the present invention, since an ultra-small motor is built in a first phalanx of a prosthetic finger, the demands of partial hand amputees of which only fingers are amputated can be satisfied.

According to the present invention, since a reducer is built between a first joint and a second joint, the first joint and the second joint can be synchronized to perform joint movements.

According to the present invention, since a cable operates by being disposed in contact with a first outer circumferential surface of a first joint and a second outer circumferential surface of a second joint, the tension or shrinkage of the cable in a longitudinal direction of the cable can be prevented.

According to the present invention, unlike the conventional technology, since a motor and reducer structure are divided and disposed in two different phalanxes of a finger, an electric prosthetic hand having a length more similar to a length of a human finger can be provided.

## Claims

1. A prosthetic finger comprising:
a first phalanx (100) having a shape corresponding to a shape from a distal phalanx to a middle phalanx of a finger; and
a motor (M) built in the first phalanx (100).

2. The prosthetic finger of claim 1, further comprising:
a second phalanx (200) having a shape corresponding to a phalanx of the finger connected to a palm; and
a first joint (110) which connects one end of the second phalanx (200) and the other end of the first phalanx (100) and receives power from the motor (M) to perform a first joint movement.

3. The prosthetic finger of claim 2, further comprising:
a reducer (G) built in the second phalanx (200); and
a second joint (210) which is formed at the other end of the second phalanx (200) and receives the power from the motor (M) to performs a second joint movement,
wherein the first joint movement and the second joint movement are synchronized with each other by the reducer (G).

4. The prosthetic finger of claim 3, further comprising a cable (C) which operates by being disposed in contact with a first outer circumferential surface (S1) of the first joint (110) positioned above a first rotary shaft (Z1) of the first joint (110) and a second outer circumferential surface (S2) of the second joint (210) positioned below a second rotary shaft (Z2) of the second joint (210) to transmit the power of the motor (M) to the first joint (110) and the second joint (210) and offset a tensile force caused by the joint movements of the first joint (110) and the second joint (210).

5. The prosthetic finger of claim 1, wherein a gearbox for performing deceleration is built in the motor (M).
